# EUROPEAN PATENT SPECIFICATION

(11) **EP 3 262 184 B1**
(45) Date of publication and mention of the grant of the patent: **02.10.2019**
(21) Application number: 15823584.6
(22) Date of filing: 16.12.2015
(51) Int. Cl.: C12Q 1/68

(54) **METHOD FOR VERIFICATION OF PRODUCT AUTHENTICITY**
VERFAHREN ZUR VERIFIZIERUNG DER PRODUKTAUTHENTIZITÄT
PROCÉDÉ POUR LA VÉRIFICATION DE L'AUTHENTICITÉ DE PRODUITS

(30) Priority: 26.02.2015 CZ 20150140
(43) Date of publication of application: 03.01.2018
(73) Proprietor: Univerzita Palackého v Olomouci, 771 47 Olomouc (CZ)
(72) Inventor: RANC, Vaclav, 783 16 Olomouc (CZ); ZBORIL, Radek, 779 00 Olomouc (CZ)
(74) Representative: Hartvichova, Katerina
(86) International application number: PCT/CZ2015/050015
(87) International publication number: WO 2016/134680

(56) References cited:
- WO-A1-2014/019099
- WO-A2-2011/116402
- US-A- 6 127 120
- US-A1- 2009 075 261
- US-A1- 2009 298 197
- US-A1- 2014 224 673
- GONÇALO DORIA ET AL: "Noble Metal Nanoparticles for Biosensing Applications", SENSORS, vol. 12, no. 12, 7 February 2012 (2012-02-07), pages 1657-1687, XP055227245, DOI: 10.3390/s120201657

### SEQUENCE LISTING

<110> Univerzita Palackeho
<120> System and method for verification of product authenticity
<130> P1396PC00
<160> 2
<170> PatentIn version 3.5
<210> 1
   <211> 40
   <212> DNA
   <213> Artificial
<220>
   <223> artificial sequence - DNA lock
<400> 1
   aaaaaggcta tacacaccag gtaacacaca cataatagcc 40
<210> 2
   <211> 33
   <212> DNA
   <213> Artificial
<220>
   <223> artificial sequence - DNA key
<400> 2
   tatgtgtgtg ttacctggtg tgtccgtgaa aaa 33

## Description

### Field of Art

The present invention relates to a method for verification of product authenticity using tagging with DNA sequences carrying SERS-active molecules.

### Background Art

Effective authenticity control of commercially available products represents a key issue in the protection of producer's rights. Its rigorous assurance leads also to the protection of consumers from potentially dangerous counterfeits which in many cases do not meet the strict requirements laid down for the original product. In principle, the authenticity protection can be ensured by several basic approaches that differ not only in their embodiment, but also in the level of protection provided. For example, a product can be protected by protective symbols placed on its package, on the basis of its unique physical properties, or by its unique chemical composition. Ideally, it is a combination of the aforementioned approaches. However, each of them has its limitations, which substantially restrict its use. This is particularly the case, where falsificators are able to produce very reliable copies of the packaging or very accurately copy the physical and chemical properties of the original, so that the counterfeited product cannot be clearly distinguished from the original, and therefore the potential violation of valid legislation and rights of the producer cannot be detected.

Other possibility of commercial product authenticity control is to add a suitable tag, a unique property said tag being monitored subsequently during the control. Authenticity control based on specific DNA addition into a product is one of the very frequent choices. This approach is described for example in WO 2000/044891, where the authors describe an ink tagged with a DNA molecule, which is then detected using molecular biology methods. The use of a complete DNA molecule has, however, certain limitations, such as the existence of an easy procedure for falsification of the tagged product. An improved modification of such method is use of polymorphic DNA fragments (US 2003/0235836), utilizing classical molecular biology methods. A significant advantage of the above mentioned modification is the possibility to use more than one polymorphic fragment, which prevents uncovering the structure of the used tag.

A significant problem of authenticity control methods based on tagging of product with DNA fragments, is the need for rapidity, simplicity and last but not least the mobility of the methods, by which the products are controlled. The often used molecular biology methods, for example PCR (polymerase chain reaction) or variable techniques of PAGE (polyacrylamide gel electrophoresis), are, despite their high popularity, often in many cases not sufficiently complying with these criteria. Other approaches based on detection of DNA, a DNA fragment or even a plurality of DNA fragments were designed, using Raman spectroscopy and recently also Surface-Enhanced Raman Scattering (SERS). Such methods are described in 2008/0189066, CN 1302905. Main advantages of this technique are its rapidity, mobility and a very low detection limit. Detection limits of units of nanomols (nmol.L⁻¹, 1×10⁻⁹ mol.L⁻¹) were already published in this domain.

In the past, the use of Raman spectroscopy in this field was limited mainly to the control of chemical composition of products, and to comparison of the original product with the potentially falsified sample. US 2008/0189066 uses Raman spectroscopy to detect products tagged with DNA fragments by analysis of said fragments in their unchanged form. A significant added value comes from WO 2014/019099, using detection of a DNA fragment obtained by DNA isolation from any organism including human DNA, animal DNA, bacterial DNA, etc., immobilized on metal nanoparticles.

Raman spectroscope is, in principle, composed of a source of light (usually a laser), which is focused on the analyzed sample. During irradiation of the sample, an effect occurs, which is called the inelastic scattering. The thus produced light is then detected and converted by a system of electronic elements into an electric signal, which is then presented as a dependence of light intensity captured by the detector on its wavelength or wavenumber, respectively. In order to significantly decrease the detection limit of such method, surface enhanced Raman scattering (SERS) phenomenon is used. This phenomenon appears during interaction of the examined molecule with noble metal nanoparticles; mainly silver and gold. The size and morphology of the particles influence the total degree of enhancement of the signal detected. In most cases, spherical nanoparticles of a diameter between 5 and 200 nm are used.

DNA fragment analysis using the SERS method allows to use very low concentrations of fragments. However, at low concentrations it is necessary to ensure the specificity of the method, in order to eliminate the risk of false positive results. There also still remains the necessity to increase the reliability and rapidity of the method. It is necessary to eliminate the influence of chemical composition of the tagged product on the results, and, at the same time, to incorporate a mechanism of internal control of the correct functioning of the whole procedure.

US 2009/0298197 discloses an assay for optical detection of bioagents, which relies on one SERS marker. If the desired sequence is present, the SERS signal is quenched. However, the system does not provide for a control whether the quenching of the SERS signal is not caused by other circumstances.

US 2009/075261 discloses a method for verification of pharmaceutical products, using nucleic acid tags with optical reporters, and relying on optical reporting and nucleic acid sequencing.

### Disclosure of the Invention

The object of the present invention is a method for verification of product authenticity, wherein
- a first DNA strand, preferably in a concentration range of from 1 fmol.L⁻¹ to 1 µmol.L⁻1, said first DNA strand containing a first molecule active in Surface-Enhanced Raman Scattering, selected from the group consisting of Cy3, Cy5, Cy7, biotin, ROX, rhodamine 6G, HEX, FAM, TET, TAMRA, bound in a region of at least one of the ends of the DNA strand, is added into the product during its production or subsequently,
- for verification of product authenticity, a second DNA strand is added into the product, said second DNA strand containing a functional group containing sulphur or nitrogen atom in the region of the first end of the DNA strand, and said second DNA strand containing a second molecule active in Surface-Enhanced Raman Scattering, selected from the group consisting of Cy3, Cy5, Cy7, biotin, ROX, rhodamine 6G, HEX, FAM, TET, TAMRA, in the region of the second end of the second DNA strand, and wherein the second DNA strand is bound to the noble metal nanoparticle surface via the functional group containing sulphur or nitrogen atom, and
wherein the second molecule active in Surface-Enhanced Raman Scattering is different from the first molecule active in Surface-Enhanced Raman Scattering, and
wherein the first DNA strand and the second DNA strand are mutually complementary in at least one part of their length, and
wherein the first and the second molecule active in Surface-Enhanced Raman Scattering are bound either both to the 5'-ends, or both to the 3'-ends of the first and the second DNA strands, and
wherein the term "in a region of one end" means that the corresponding molecule and/or a functional group is bound to one of the last 5 nucleotides of the corresponding end of the DNA strand,
- subsequently, the hybridization product formed by interaction of the first and the second DNA strand is isolated,
- spectrum of the isolated hybridization product is measured using Surface-Enhanced Raman Scattering method.

In a preferred embodiment, the noble metal nanoparticles are in the form of a composite containing at least one magnetic particle (preferably a nanoparticle) and at least one noble metal nanoparticle. The presence of the magnetic particle enables to easily separate the composite with bound groups by methods of magnetic separation. Suitable magnetic particles are for example iron oxide nanoparticles.

The molecule active in Surface-Enhanced Raman Scattering is selected from Cy3, Cy5, Cy7, biotin, ROX, rhodamine 6G, HEX, FAM, TET, and TAMRA. The Surface-Enhanced Raman Scattering active molecule should be selected so that its Raman spectrum (SERS) would be different from the spectrum of the DNA strand. Preferably, the Surface-Enhanced Raman Scattering active molecule is covalently bound to the DNA strand.

The functional group containing sulphur or nitrogen atom ensures binding of the second DNA strand to the nanoparticle surface. It may be in particular a functional group containing dithiol, thiol, carboxyl, amino or nitro group.

Preferably, the noble metal is gold or silver.

Preferably, noble metal nanoparticles have the size from 5 to 100 nm.

When the second end of the second DNA strand, where the second Surface-Enhanced Raman Scattering active molecule is bound, is the 5'-end, then the first Surface-Enhanced Raman Scattering active molecule is bound also to the 5'-end of the first DNA strand. Accordingly, in another embodiment, when the second end of the second DNA strand, where the second Surface-Enhanced Raman Scattering active molecule is bound, is the 3'-end, then the first Surface-Enhanced Raman Scattering active molecule is bound also to the 3'-end of the first DNA strand.

The second DNA strand contains preferably at least 20 nucleotides.

The first DNA strand contains preferably at least from 5 to 40 nucleotides.

The first DNA strand and the second DNA strand are mutually complementary in at least 5 % of their length, preferably in at least 20 % of their length, more preferably in at least 50 % of their length.

The term "in a region of one end" means that the corresponding molecule and/or a functional group is bound to one of the last 5 nucleotides of the corresponding end of the DNA strand, preferably to one of the last 3 nucleotides, most preferably to the last nucleotide.

In a preferred embodiment, the second DNA strand forms loop-shaped structures on the nanoparticle surface. The loop-shaped structures begin and also end on the nanoparticle surface, which enables, in the absence of the first DNA strand, the second Surface-Enhanced Raman Scattering active molecule to interact with the nanoparticle surface.

Preferably, the first and second DNA strands are selected so that they are neither identical nor similar to any DNA present in the analyzed product.

The acquired spectrum of the isolated hybridization product is then compared to reference spectra, which are preferably spectra of the second DNA strand containing in a region of the first end of the DNA strand a functional group containing sulphur or nitrogen atom, and in a region of the second end of the DNA strand a second molecule active in Surface-Enhanced Raman Scattering. Reference spectra can also be spectra of the analyzed product without addition of the first DNA strand with a first molecule active in Surface-Enhanced Raman Scattering, and spectra of the analyzed product containing the first DNA strand with a first molecule active in Surface-Enhanced Raman Scattering.

If the spectrum of the isolated hybridization product shows lower Raman signal intensity of the lock (mainly the second molecule active in Surface-Enhanced Raman Scattering) and at the same time Raman signal of the key (mainly the first molecule active in Surface-Enhanced Raman Scattering) appears in the spectrum, it proves the presence of the first DNA strand in the analyzed product, and therefore the authenticity of the product.

In one preferred embodiment, the noble metal nanoparticles are in the form of a composite containing at least one magnetic particle (preferably a nanoparticle) and at least one noble metal nanoparticle, and the isolation of the hybridization product is performed using methods of magnetic isolation.

In another preferred embodiment, after the addition of the second DNA strand into the product and before the hybridization product isolation step, the mixture is allowed to react for 1 to 7200 seconds.

The principle of verification of product authenticity using the method according to the present invention is based on the fact that the product contains only a very small amount of the first DNA strand, containing a first molecule active in Surface-Enhanced Raman Scattering (key). During the authenticity verification, the second DNA strand, containing in a region of the first end of the DNA strand a functional group containing sulphur or nitrogen atom, and in a region of the second end of the DNA strand a second molecule active in Surface-Enhanced Raman Scattering, whereas the second DNA strand is bound to the surface of a noble metal nanoparticle (the whole composite is called "lock") via its functional group containing sulphur or nitrogen atom, is added to the product, whereas the second molecule active in Surface-Enhanced Raman Scattering is different from the first molecule active in Surface-Enhanced Raman Scattering.

As long as the second DNA strand (lock) is not in contact with the first DNA strand (key), the second molecules active in Surface-Enhanced Raman Scattering can be in the proximity of noble metal nanoparticles, and therefore their Raman signal, enhanced by this interaction, can be detected. The authentic product is characterized in that it contains the first DNA strand with first molecules active in Surface-Enhanced Raman Scattering. The sequences of the first and the second DNA strand are mutually complementary in at least one part of their length. When they get into contact with each other, hybridization occurs, the second DNA strand straightens, the second molecules active in Surface-Enhanced Raman Scattering move away from the noble metal nanoparticle surface, while the first molecules active in Surface-Enhanced Raman Scattering get into the proximity of the noble metal nanoparticle surface, due to the hybridization of the DNA strands. This causes decrease or even a complete suppression of the Raman signal of the lock, i.e. of the second molecules active in Surface-Enhanced Raman Scattering (depending on their concentration ratio), and to appearance of the Raman signal of the key, i.e. of the first molecules active in Surface-Enhanced Raman Scattering. If the product is not authentic, the first DNA strands with the first molecules active in Surface-Enhanced Raman Scattering are not present therein, therefore the above described changes in the spectra do not occur. The measured spectrum shows again only the signal of the second molecules active in Surface-Enhanced Raman Scattering (spectrum of the lock).

When the composite with magnetic particles contains also noble metal nanoparticles, the method is facilitated because the hybridization product (or the lock itself in the case of non-authentic product) can be easily separated using magnetic methods, thus preventing non-specific interactions of sample components with the lock from interfering with the measurement.

### Brief Description of Drawings

Fig. 1 depicts graphically the principle of product tagging with the key (first DNA strand) and of the analysis using the lock (second DNA strand).
Fig. 2 depicts an example of spectra obtained from product (distilled water) analysis without addition of the tag (full line) and with addition of the tag (dotted line) according to Example 2.
Fig. 3 depicts an example of spectra obtained from product (whiskey) analysis without addition of the tag (full line) and with addition of the tag (dotted line) according to Example 3.

### Examples

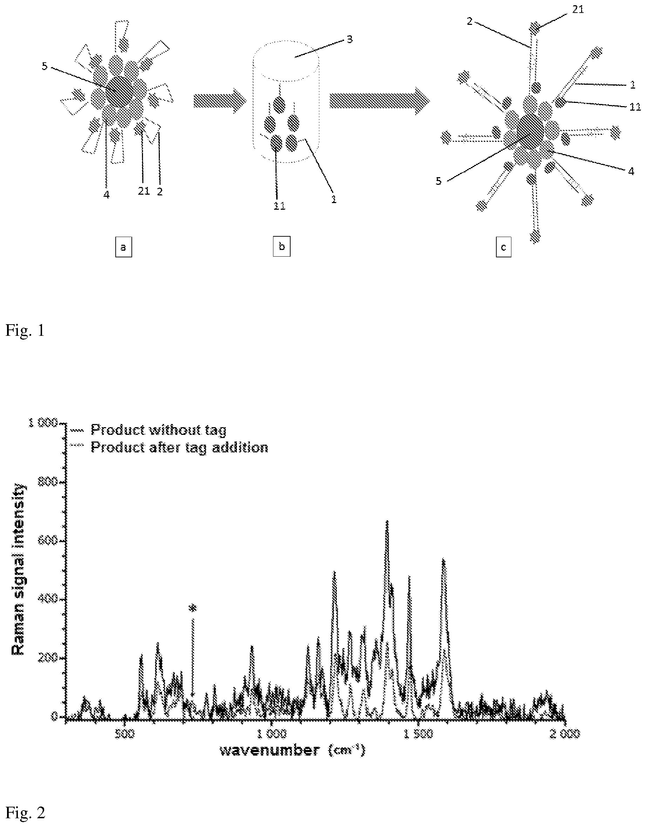

Example 1 - *Explanation of the principle of the invention using a schematic representation* Fig. 1 depicts schematically the principle of product tagging using a key (first DNA strand) and of the analysis using a lock (second DNA strand). Figure a) depicts the lock itself before its interaction with a key. The lock contains a second DNA strand 2 carrying in its first end region of the DNA strand a second molecule 21 active in Surface-Enhanced Raman Scattering. In the region of its second end, it is bound via a functional group (not depicted) to a noble metal nanoparticle 4.

The noble metal nanoparticles 4 are in the form of a composite with magnetic nanoparticles 5. Second DNA strands 2 form loops, so that second molecules 21 interact with noble metal nanoparticles 4, therefore measurement of the lock gives SERS spectra of the second molecules 21.

Figure b) depicts a product 3, which contains keys - first DNA strands 1, containing in the region of one of their ends first molecules 11 active in Surface-Enhanced Raman Scattering.

Locks are added into a sample of the product 3 containing keys, and a hybridization product, depicted in figure c), is formed. Hybridization of the first and the second DNA strands 1 and 2 occurs, which moves away second molecules 21 active in Surface-Enhanced Raman Scattering from noble metal nanoparticles 4, therefore their SERS signal is suppressed. On the contrary, first molecules 11 active in Surface-Enhanced Raman Scattering, get into contact with noble metal nanoparticles 4, and therefore spectra of the first molecules 11 are obtained from measurements of the hybridization product.

### Example 2 - Lock and key nanocomposite preparation, lock - key interaction, SERS spectra measurements

Pure nanocomposite of noble metal nanoparticles with magnetic nanoparticles of Ag/Fe₃O₄ was prepared based on the method described in Marková, Z., Š̌̌iš̌̌ková, K., Filip, J., Š̌afářová, K., Prucek, R., Panáček, A., Zbořil, R. et al. (2012). Chitosan-based synthesis of magnetically-driven nanocomposites with biogenic magnetite core, controlled silver size, and high antimicrobial activity. Green Chemistry, 14(9), 2550.

Nanocomposite sample was diluted 5 times and measured using Raman spectroscopy. 70 µL of distilled water and 10 µL of DNA lock (eg. second DNA strand carrying a functional group and a second molecule active in SERS) of the sequence 5'-[DTPA]AAAAAGGCTATA*CACACCAGGTAACACACACA*TAATAGCC[Cy3]-3' were gradually added into 20 µL of nanocomposite solution. DTPA = diethylene triamine pentaacetic acid, a functional group carrying amine groups. The resulting DNA concentration used for the modification was 10 nmol/L. The mixture was then kept at room temperature for 10 minutes in order to create the lock structure. Subsequently, the mixture was used for product authenticity control.

The key - first DNA strand carrying first a molecule active in SERS - was added into a sample of distilled water (representing a sample of the product). The key sequence was: 5'-*TATGTGTGTGTTACCTGGTGTGT*CCGTGAAAAA - biotin-3'. The final DNA key concentration was 10 nmol.L⁻¹. Italics in both sequences stand for the complementary parts. 10 µL of the lock containing mixture was then added into 1 mL of product. After mixing, the resulting hybridization product was magnetically isolated and washed three times with distilled water.

The following key detection is based on spectral analysis of the isolated hybridization product using Surface-Enhanced Raman Scattering and on statistical comparison of the hybridization product spectrum with the spectrum acquired from pure lock analysis (by method of discrimination analysis).

Adjusting of experimental SERS parameters depends on individual properties and configuration of the apparatus used. In the above mentioned example, the sample was irradiated using 532 nm wavelength laser, the power of which was set to 10 mW by inserted optical elements. The exposition time was adjusted to 1 second, and the total of 32 measurements were performed. The resulting spectrum is the average of all measurements. If the key is present in the analysed product (the product is authentic), the spectra of DNA lock are reduced, while key signals appear (marked with *). Example of resulting spectra is depicted in Fig. 2. If the analysed product is not authentic (key is not present), the resulting spectra do not contain key signals and the lock signals stay unchanged.

### Example 3 - Using the method according to the present invention in a real product sample (whiskey)

The lock and the key were of the same structure as in Example 2. The key was added to a sample of whiskey, its final concentration was 10 nmol/L.

Subsequently, 10 µL of a lock containing mixture was added into 1 mL of sample of whiskey. After vigorous mixing, the hybridization product was magnetically isolated and washed three times with distilled water.

Key detection was based on spectral analysis of the isolated hybridization product using Surface-Enhanced Raman Scattering and on statistical comparison of the hybridization product spectrum with the spectrum acquired from pure lock analysis (by method of discrimination analysis).

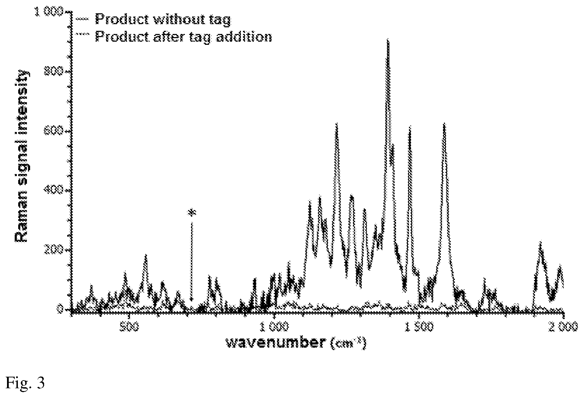

Magnetically isolated and washed hybridization product underwent spectral analysis using Raman spectroscopy. Adjusting of experimental SERS parameters depends on individual properties and configuration of the apparatus used. In this example, the sample was irradiated using 532 nm wavelength laser, the power of which was set to 10 mW by inserted optical elements. The exposition time was adjusted to 1 second, and the total of 32 measurements were performed. The resulting spectrum is the average of all measurements. If the key is present in the analysed product (the product is authentic), the spectra of DNA lock are reduced, while key signals appear (marked with *). Example of resulting spectra is depicted in Fig. 3. If the analysed product is not authentic (key is not present), the resulting spectra do not contain key signals and the lock signals stay unchanged.

## Claims

1. A method for verification of product authenticity, wherein
- a first DNA strand, preferably in a concentration range of from 1 fmol.L⁻¹ to 1 µmol.L⁻¹, said first DNA strand containing a first molecule active in Surface-Enhanced Raman Scattering, selected from the group consisting of Cy3, Cy5, Cy7, biotin, ROX, rhodamine 6G, HEX, FAM, TET, TAMRA, bound in a region of at least one of the ends of the DNA strand, is added into the product during its production or subsequently,
- for verification of product authenticity, a second DNA strand is added into the product, said second DNA strand containing a functional group containing sulphur or nitrogen atom in the region of the first end of the DNA strand, and said second DNA strand containing a second molecule active in Surface-Enhanced Raman Scattering, selected from the group consisting of Cy3, Cy5, Cy7, biotin, ROX, rhodamine 6G, HEX, FAM, TET, TAMRA, in the region of the second end of the second DNA strand, and wherein the second DNA strand is bound to a noble metal nanoparticle surface via the functional group containing sulphur or nitrogen atom,
and
wherein the second molecule active in Surface-Enhanced Raman Scattering is different from the first molecule active in Surface-Enhanced Raman Scattering, and
wherein the first DNA strand and the second DNA strand are mutually complementary in at least one part of their length, and
wherein the first and the second molecule active in Surface-Enhanced Raman Scattering are bound either both to the 5'-ends, or both to the 3'-ends of the first and the second DNA strands, and
wherein the term "in a region of one end" means that the corresponding molecule and/or a functional group is bound to one of the last 5 nucleotides of the corresponding end of the DNA strand,
- subsequently, the hybridization product formed by interaction of the first and the second DNA strand is isolated,
- spectrum of the isolated hybridization product is measured using Surface-Enhanced Raman Scattering method.

2. The method according to claim 1, wherein the noble metal nanoparticles are in the form of a composite containing at least one magnetic particle, preferably a nanoparticle, and at least one noble metal nanoparticle.

3. The method according to any one of the preceding claims, wherein the functional group contains dithiol, thiol, carboxyl, amino or nitro group.

4. The method according to any one of the preceding claims, wherein the noble metal is gold or silver.

5. The method according to any one of the preceding claims, wherein the noble metal nanoparticles have the size from 5 to 100 nm.

6. The method according to any one of the preceding claims, wherein the second DNA strand contains at least 20 nucleotides, and the first DNA strand contains at least from 5 to 40 nucleotides.

7. The method according to any one of the preceding claims, wherein the second DNA strand on the nanoparticle surface is adapted for formation of loops, which begin and end on the nanoparticle surface.

8. The method according to claim 1, wherein the noble metal nanoparticles are in the form of a composite containing at least one magnetic particle, preferably a nanoparticle, and at least one noble metal nanoparticle, and the isolation of the hybridization product is performed using methods of magnetic isolation.

9. The method according to any one of the preceding claims, wherein after the addition of second DNA strand into the product and before the hybridization product isolation step, the mixture is allowed to react for 1 to 7200 seconds.

## Patentansprüche

1. Verfahren zur Verifizierung der Produktauthentizität, wobei
- einen ersten DNA-Strang, vorzugsweise in einem Konzentrationsbereich von 1 fmol.L⁻¹ bis 1 µmοl.L⁻¹, wobei der erste DNA-Strang ein erstes Molekül enthält, das in der oberflächenverstärkten Raman-Streuung aktiv ist und ausgewählt aus der Gruppe bestehend aus Cy3, Cy5, Cy7, Biotin, ROX, Rhodamin 6G, HEX, FAM, TET, TAMRA ist, gebunden in einem Bereich von mindestens einem der Enden des DNA-Strangs, wird dem Produkt während seiner Herstellung oder anschließend zugesetzt,
- zur Verifizierung der Produktauthentizität wird dem Produkt ein zweiter DNA-Strang zugesetzt, der eine funktionelle Gruppe, die ein Schwefel- oder Stickstoffatom enthält, im Bereich des ersten Endes des DNA-Strangs, und wobei der zweite DNA-Strang enthält ein zweites Molekül, das in der oberflächenverstärkten Raman-Streuung aktiv ist und ausgewählt aus der Gruppe bestehend aus Cy3, Cy5, Cy7, Biotin, ROX, Rhodamin 6G, HEX, FAM, TET, TAMRA ist, im Bereich des zweiten Endes des zweiten DNA-Strangs, und wobei der zweite DNA-Strang über die Schwefel- oder Stickstoffatom enthaltende funktionelle Gruppe an eine Edelmetallnanopartikeloberfläche gebunden ist, und wobei sich das zweite Molekül, das bei der oberflächenverstärkten Raman-Streuung aktiv ist, unterscheidet von dem ersten Molekül, das bei der oberflächenverstärkten Raman-Streuung aktiv ist,
und
wobei der erste DNA-Strang und der zweite DNA-Strang in mindestens einem Teil ihrer Länge zueinander komplementär sind, und
wobei das erste und das zweite Molekül, die bei der oberflächenverstärkten Raman-Streuung aktiv sind, entweder beide an die 5'-Enden oder beide an die 3'-Enden des ersten und des zweiten DNA-Strangs gebunden sind, und
wobei der Begriff "im Bereich eines Endes" bedeutet, dass das entsprechende Molekül und/oder die funktionelle Gruppe an eines der letzten 5 Nukleotide des entsprechenden Endes des DNA-Strangs gebunden ist,
- anschließend das durch Wechselwirkung des ersten und des zweiten DNA-Strangs gebildete Hybridisierungsprodukt isoliert wird,
- Das Spektrum des isolierten Hybridisierungsprodukts wird mit der Methode der oberflächenverstärkten Raman-Streuung gemessen.

2. Verfahren nach Anspruch 1, **dadurch gekennzeichnet, dass** die Edelmetallnanopartikel in Form eines Komposits vorliegen, das mindestens ein magnetisches Partikel, vorzugsweise ein Nanopartikel, und mindestens ein Edelmetallnanopartikel enthält.

3. Verfahren nach einem der vorhergehenden Ansprüche, wobei die funktionelle Gruppe eine Dithiol-, Thiol-, Carboxyl-, Amino- oder Nitrogruppe enthält.

4. Verfahren nach einem der vorhergehenden Ansprüche, wobei das Edelmetall Gold oder Silber ist.

5. Verfahren nach einem der vorhergehenden Ansprüche, wobei die Edelmetallnanopartikel die Größe von 5 bis 100 nm aufweisen.

6. Verfahren nach einem der vorhergehenden Ansprüche, wobei der zweite DNA-Strang mindestens 20 Nukleotide enthält und der erste DNA-Strang mindestens 5 bis 40 Nukleotide enthält.

7. Verfahren nach einem der vorhergehenden Ansprüche, wobei der zweite DNA-Strang auf der Nanopartikeloberfläche zur Bildung von Schleifen angepasst ist, die auf der Nanopartikeloberfläche beginnen und enden.

8. Verfahren nach Anspruch 1, wobie die Edelmetallnanopartikel in Form eines Komposits vorliegen, das mindestens ein magnetisches Partikel, vorzugsweise ein Nanopartikel, und mindestens ein Edelmetallnanopartikel enthält, und die Isolierung des Hybridisierungsprodukts unter Verwendung von Methoden der magnetischen Isolierung.

9. Verfahren nach einem der vorhergehenden Ansprüche, wobei nach der Zugabe des zweiten DNA-Strangs in das Produkt und vor dem Hybridisierungsprodukt-Isolierungsschritt das Gemisch 1 bis 7200 Sekunden lang reagieren gelassen wird.

## Revendications

1. Procédé pour la vérification de l'authenticité du produit, dans laquelle
- un premier brin d'ADN, de préférence dans une gamme de concentrations allant de 1 fmol.L⁻¹ à 1 µmοl.L⁻¹, ledit premier brin d'ADN contenant une première molécule active dans la diffusion Raman améliorée en surface et étant choisi dans le groupe constitué de Cy3, Cy5, Cy7, biotine, ROX, rhodamine 6G, HEX, FAM, TET, TAMRA, liés dans une région d'au moins une des extrémités du brin d'ADN, est ajouté au produit lors de sa production ou ultérieurement,
- pour la vérification de l'authenticité du produit, un deuxième brin d'ADN est ajouté au produit, ledit deuxième brin d'ADN contenant un groupe fonctionnel contenant un atome de soufre ou d'azote dans une région de la première extrémité du brin d'ADN et ledit deuxième brin d'ADN contenant une deuxième molécule active dans la diffusion Raman améliorée en surface, sélectionnée dans le groupe constitué de Cy3, Cy5, Cy7, biotine, ROX, rhodamine 6G, HEX, FAM, TET, TAMRA, dans une région de la seconde extrémité du deuxième brin d'ADN, et où le second brin d'ADN est lié à une surface de nanoparticule de métal noble via le groupe fonctionnel contenant un atome de soufre ou d'azote, et
où la deuxième molécule active dans la diffusion Raman améliorée en surface est différente de la première molécule active dans la diffusion Raman améliorée en surface, et
où le premier brin d'ADN et le deuxième brin d'ADN sont mutuellement complémentaires dans au moins une partie de leur longueur, et
où la première et la deuxième molécules actives dans la diffusion Raman améliorée en surface sont liées à la fois aux extrémités 5', ou aux extrémités 3' des premier et deuxième brins d'ADN, et
où le terme "dans une région d'une extrémité" signifie que la molécule correspondante et/ou un groupe fonctionnel correspondant est lié(e) à l'un des 5 derniers nucléotides de l'extrémité correspondante du brin d'ADN,
- ensuite, le produit d'hybridation formé par interaction du premier et du deuxième brin d'ADN est isolé,
- le spectre du produit d'hybridation isolé est mesuré à l'aide de la méthode de diffusion Raman en surface améliorée.

2. Procédé selon la revendication 1, dans lequel les nanoparticules de métal noble se présentent sous la forme d'un composite contenant au moins une particule magnétique, de préférence une nanoparticule, et au moins une nanoparticule de métal noble.

3. Procédé selon l'une quelconque des revendications précédentes, dans lequel le groupe fonctionnel contient un groupe dithiol, thiol, carboxyle, amino ou nitro.

4. Procédé selon l'une quelconque des revendications précédentes, dans lequel le métal noble est l'or ou l'argent.

5. Procédé selon l'une quelconque des revendications précédentes, dans lequel les nanoparticules de métal noble ont une taille de 5 à 100 nm.

6. Procédé selon l'une quelconque des revendications précédentes, dans lequel le deuxième brin d'ADN contient au moins 20 nucléotides et le premier brin d'ADN contient au moins 5 à 40 nucléotides.

7. Procédé selon l'une quelconque des revendications précédentes, dans lequel le deuxième brin d'ADN sur la surface de nanoparticules est adapté pour la formation de boucles qui commencent et finissent sur la surface de nanoparticules.

8. Procédé selon la revendication 1, dans lequel les nanoparticules de métal noble se présentent sous la forme d'un composite contenant au moins une particule magnétique, de préférence une nanoparticule, et au moins une nanoparticule de métal noble, et l'isolement du produit d'hybridation est effectué à l'aide de méthodes d'isolation magnétique.

9. Procédé selon l'une quelconque des revendications précédentes, dans lequel, après l'addition du second brin d'ADN dans le produit et avant l'étape d'isolement du produit d'hybridation, on laisse le mélange réagir pendant 1 à 7200 secondes.
